# EUROPÄISCHE PATENTANMELDUNG

(11) **EP 1 084 732 A2**
(43) Veröffentlichungstag der Anmeldung: **21.03.2001**
(21) Anmeldenummer: 00119212.9
(22) Anmeldetag: 06.09.2000
(51) Int. Cl.: A61N 5/06

(54) **Bräunungsliege**

(30) Priorität: 15.09.1999 DE 19944975
(71) Anmelder: Kopper, Iris, 44227 Dortmund (DE)
(72) Erfinder: Pürschel, Dieter, 44227 Dortmund (DE)
(74) Vertreter: Hemmerich, Friedrich Werner

(57) **Zusammenfassung**

Eine Bräunungsliege, die aus einer, auf einer Bodenplatte (BP) auf stehenden Liege (LG) für die zu bestrahlende Person und einem, um eine horizontale Achse schwenkbaren Bestrahlungshimmel (HM) besteht. Dieser ist aus einer, die Liege (LG) zum Einstieg freigebenden Position in eine Bestrahlungsposition schwenkbar und als Kugelabschnitt ausgebildet. Auf dessen, der Liege zugewandten, gewölbten Innenwand (3) ist eine Vielzahl von Reflektionskörpern (RK) angeordnet, die durch die Strahlen von, ausserhalb des Bestrahlungshimmels (HM), in dessen Randbereich angeordneten Bestrahlungslichtquellen (BL) beaufschlagbar sind.

## Beschreibung

Die Erfindung betrifft eine Bräunungsliege, bestehend aus einer, auf dem Boden oder einer Bodenplatte aufstehenden Liege für die zu bestrahlende Person und einem, um eine horizontale Achse schwenkbaren Bestrahlungshimmel, der aus einer, die Liege zum Einstieg freigebenden Position in eine Bestrahlungsposition oberhalb der Liege, diese teilweise umfassend, schwenkbar ist.

Bestrahlungsliegen dieser Art sind in zahlreichen Ausbildungsformen bekannt geworden, die sämtlich den gemeinsamen Nachteil aufweisen, dass sich die, auf der Liege aufliegende Person, nachdem der Bestrahlungshimmel in die Bestrahlungsposition geschwenkt wurde, in einem sehr engen, von Lüfterluft durchströmten Raum befindet, wobei die Bestrahlungslichtquellen in dem Himmel in verhältnismässig kleinen Abständen über dem Körper der Person angeordnet sind. Diese Verhältnisse werden u.a. wegen der beschränkten Bewegungsfreiheit, der Wärmebelastung und auch der Blendwirkung der Bestrahlungslichtquellen durchweg als mehr oder weniger unangenehm empfunden, besonders von Personen mit klaustrophobischen Beschwerden.

Es wurde bereits vorgeschlagen, diesen Nachteilen durch eine, nach oben offene Bauweise der Einrichtungen zu begegnen, bei denen, oberhalb der Liege in grösseren Abständen einzelne Bestrahlichtquellen angeordnet wurden. Es sind auch Vorschläge bekannt geworden, die Strahlen der Bestrahlungslichtquellen nicht direkt sondern über Umlenkspiegel auf die Liege zu richten

Der Erfindung liegt die Aufgabe zugrunde, gattungsgemässe Bräunungsliegen so zu verbessern, dass bei erheblich grösserer Bewegungsfreiheit der zu bestrahlenden Person, auch während der Bestrahlung, diese Bestrahlung im wesentlichen indirekt unter geringer Wärmebelastung bewirkt wird.

Diese Aufgabe wird dadurch gelöst, dass der Bestrahlungshimmel als Kugel- oder Zylinderabschnitt ausgebildet ist, auf dessen, der Liege zugewandter, gewölbter Innenwand ein Reflektionsbelag oder eine Vielzahl von Reflektionskörpern angeordnet sind, die durch die Strahlen von innerhalb oder ausserhalb des Bestrahlungshimmels oder/und in dessen Randbereich angeordneten Bestrahlungslichtquellen beaufschlagbar sind.

Wie die Erfindung weiter vorsieht, können die, von den Bestrahlungslichtquellen ausgehenden Lichtstrahlen gebündelt und auf bestimmte Abschnitte der gewölbten Innenwand gerichtet werden. Die Reflektionskörper können dabei aus Reihen von Spiegelplatten mit ebenen oder gewölbten Spiegelflächen bestehen. Die Spiegelplatten können winkelverstell- festlegbar angeordnet werden. Ein, den Kugel- oder Zylinderabschnitt aufnehmendes Gehäuse wird zweckmässig, beiderseits des Oeffnungsdurchmessers in horizontalen Schwenklagern gelagert.

Weiter können erfindungsgemäss die Bestrahlungslichtquellen auf einem, mit Abstand unterhalb der Oeffnungsebene des, in Bestrahlungsposition befindlichen Bestrahlungshimmels, um die Liege herumgeführten Tragrahmen in der Form eines, nach vorn offenen Kreisabschnitts angeordnet sein.

Schliesslich können Liege, Bestrahlungshimmel und Bestrahlungslichtquellen in einer, nach oben offenen Zylinderkabine angeordnet sein, die aus einer ortsfest aufstehenden, etwa halbzylindrischen Tragwand und einer, gleitend um diese oder in dieser geführten, ebenfalls etwa halbzylindrischen Schliesswand besteht.

Die Erfindung wird anhand des, in der Zeichnung dargestellten Ausführungsbeispiels naher erläutert. In der Zeichnung zeigen:
- Fig. 1: die Bräunungsliege mit Kabine, seitlich von oben gesehen, in perspektivischer Darstellung,
- Fig. 2: die Draufsicht auf Fig.2 ohne Bräunungsliege,
- Fig. 3: die Vorderansicht von Fig. 1 in schematischer Darstellung,
- Fig. 4: die Draufsicht auf Fig.3 und
- Fig. 5: die Seitenansicht von Fig. 3, ebenfalls in schematischer Darstellung.

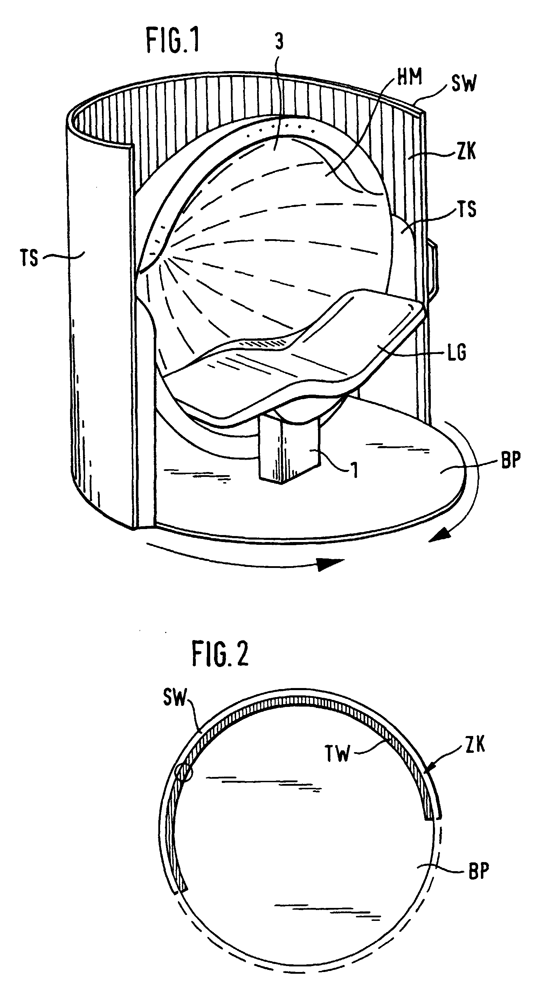

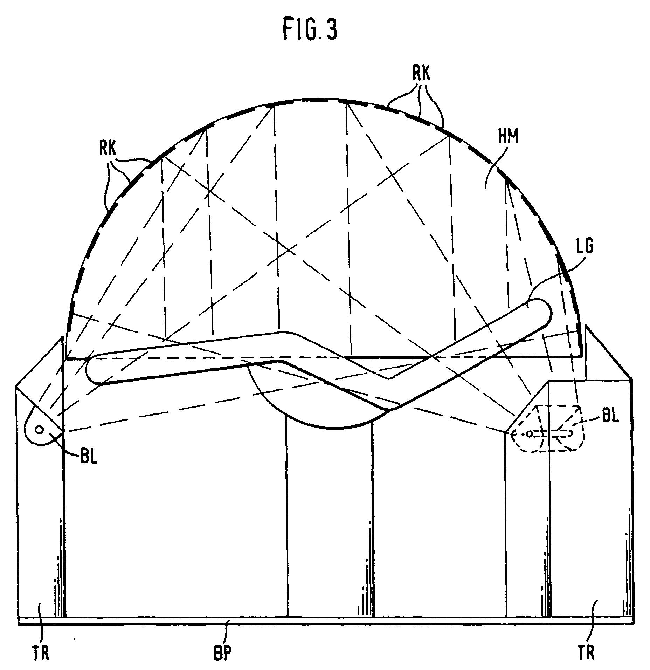

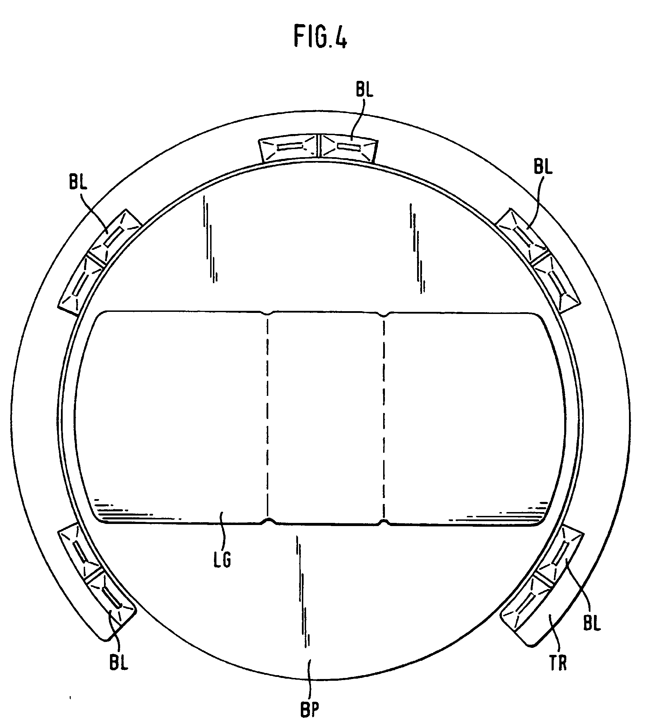

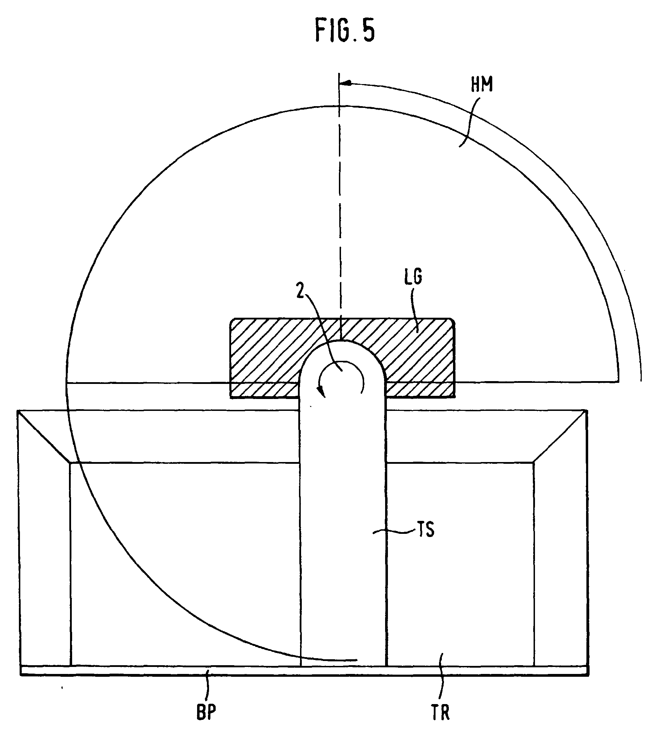

Wie aus Fig. 1 zu ersehen, ist die, auf nicht dargestellte Weise heb-, senk- und schwenkbar ausgebildete Liege LG mit ihrem Fuss 1 auf einer Bodenplatte BP angeordnet. Diese Bodenplatte BP trägt eine, nach oben offene Zylinderkabine ZK, die aus einer, auf der Bodenplatte BP aufstehenden, etwa halbzylindrischen Tragwand TW und einer, um diese herum, gleitschienengeführten, etwa halbzylindrischen Schliesswand SW besteht. Innerhalb der halbzylindrischen Tragwand TW sind, mit Abstand von den beiden Enden der Liege LG diametral einander gegenüber, auf der Bodenplatte BP aufstehende Tragstützen TS angeordnet, zwischen denen, in Schwenklagern 2 (verl. Fig. 5) der Bestrahlungshimmel HM schwenkgelagert ist, der hier eine Halbkugel bildet. Der Bestrahlungshimmel HM ist aus der, in Fig.1 wiedergegebenen offenen Einstiegsposition, bei der die kreisförmige Oeffnung in einer etwa senkrechten Ebene liegt, in die, in Fig. 5 wiedergegebene Schliessposition schwenkbar, bei der die Oeffnung in einer etwa horizontalen Ebene liegt und die Liege LG umschliesst. Mit Abstand unterhalb der Oeffnung in deren horizontaler Lage, ist ein kreisförmig um die Liege LG und die Oeffnung herum geführter, vorne offener Tragrahmen TR angeordnet, der hier, auf der Bodenplatte BP aufstehend, an der Tragwand anliegt (Fig. 3 und 4). Am oberen Rand dieses Tragrings TR sind, auf nicht dargestellte Weise richtungsverstell- und festlegbare Bestrahlungslichtquellen BL mit Abstand voneinander, ringförmig angeordnet. In der gewölbten Innenwand 3 des Bestrahlungshimmels HM ist eine Vielzahl von Reflektionskörpern RF eingesetzt, die in vorberechnete Reflektions-Winkelpositionen eingebracht, die Möglichkeit schaffen, wie aus Fig. 3 zu ersehen, die von den Bestrahlungslichtquellen BL ausgehenden Strahlen richtungsgezielt und ggfs. auch vorberechnet gebündelt, auf bestimmte Abschnitte der Liege LG bzw. auf entsprechende Körperflächen der zu bestrahlenden Person zu richten. Anstelle dieser Reflektionskörper kann auch die gesamte Innenwand 3 mit einem Reflektionsbelag versehen werden.

Die Zylinderkabine ZK kann in der geöffneten Stellung nach Fig. 1, bei der die Schliesswand SW zurückgeschoben und der Bestrahlungshimmel HM zurückgeschwenkt worden ist, ohne Behinderung durch seitliche Wand- oder andere Einrichtungsteile betreten werden. Die, zu bestrahlende Person kann nach dem Eintritt, ebenfalls unbehindert die Liege LG in die gewünschte Lage kippen, höheneinstellen und aur der Liege platznehmen, unbehindert durch den Bestrahlungshimmel HM.

Nach Schliessen der Schliesswand SW, ggfs. auch ohne diese und Schwenken des Bestrahlungshimmles HM in Bestrahlungsposition bleibt, vor allen Dingen über der Liege LG viel freier Raum und die Bestrahlung erfolgt fast ohne Blendwirkung und unter geringer Wärmebelastung.

### Bezugszeichenaufstellung

- 1: Fuss
- 2: Schwenklager
- 3: (gewölbte) Innenwand
- LG: Liege
- BP: Bodenplatte
- ZK: Zylinderkabine
- TW: Tragwand
- TS: Tragstützen
- SW: Schliesswand
- HM: Bestrahlungshimmel
- TR: Tragrahmen
- BL: Bestrahlungslichtquellen
- RK: Reflektionskörper

## Patentansprüche

1. Bräunungsliege, bestehend aus einer, auf dem Boden oder einer Bodenplatte aufstehenden Liege für die zu bestrahlende Person und einem, um eine horizontale Achse schwenkbaren Bestrahlungshimmel, der aus einer, die Liege zum Einstieg freigebenden Position in eine Bestrahlungsposition oberhalb der Liege, diese teilweise umfassend schwenkbar ist,
**dadurch gekennzeichnet,**
dass der Bestrahlungshimmel (HM) als Kugel- oder Zylinderabschnitt ausgebildet ist, auf dessen, der Liege (LG) zugewandter gewölbter Innenwand (3) ein Reflektionsbelag oder eine Vielzahl von Reflektionskörpern (RK) angeordnet sind, die durch die Strahlen von, innerhalb oder ausserhalb des Bestrahlungshimmels (HM) oder in dessen Randbereich angeordneten Bestrahlungslichtquellen (BL) beaufschlagbar sind.

2. Bräunungsliege nach Anspruch 1,
**dadurch gekennzeichnet,**
dass die, von den Bestrahlungslichtquellen (BL) ausgehenden Lichtstrahlen bündel- und auf bestimmte Abschnitte der Kugel- bzw. Zylinderabschnittsinnenwand (3) richtbar sind.

3. Bräunungsliege nach den Ansprüchen 1 und/oder 2,
**dadurch gekennzeichnet,**
dass die Reflektionskörper (RK) aus Reihen von Spiegelplatten mit ebenen oder gewölbten Spiegelflächen bestehen.

4. Bräunungsliege nach Anspruch 3,
**dadurch gekennzeichnet,**
dass die Reflektionskörper (RK) winkelverstell- und festlegbar sind.

5. Bräunungsliege nach einem oder mehreren der Ansprüche 1 - 4,
**dadurch gekennzeichnet,**
dass der Kugel- bzw. der Zylinderabschnitt in einem Gehäuse angeordnet sind, das beiderseits des Oeffnungsdurchmesers in horizontalen Schwenklagern lagert.

6. Bräunungsliege nach Anspruch 5,
**dadurch gekennzeichnet,**
dass die Bestrahlungslichtquellen (BL) auf einem, mit Abstand innerhalb der Oeffnungsebene des, in Bestrahlungsposition befindlichen Bestrahlungshimmels (HM) um die Liege (LG) herumgeführten Tragrahmen (TR) angeordnet sind.

7. Bräunungsliege nach einem der Ansprüche 1 - 6,
**dadurch gekennzeichnet,**
dass Liege (LG) und Bestrahlungshimmel (HM) in einer, nach oben offenen Zylinderkabine angeordnet sind, die aus einer ortsfest aufstehenden etwa halbzylindrischen Tragwand (TW) und einer, gleitend um diese oder in dieser geführten, ebenfalls etwa halbzylindrischen Schliesswand (SW) besteht.
